# EUROPEAN PATENT APPLICATION

(11) **EP 4 215 119 A1**
(43) Date of publication of application: **26.07.2023**
(21) Application number: 21869691.2
(22) Date of filing: 15.09.2021
(51) Int. Cl.: A61B 10/00, C12M 1/30, G01N 1/10, A61B 10/02, G01N 1/02

(54) **SAMPLE COLLECTION STICK**

(30) Priority: 16.09.2020 KR 20200119514; 05.02.2021 KR 20210017056; 05.02.2021 KR 20210017061; 04.05.2021 KR 20210057681
(71) Applicant: Bionlifescience, Inc., Namyangju-si, Gyeonggi-do 12106 (KR)
(72) Inventor: GOH, Chang Wook, Namyangju-si Gyeonggi-do 12095 (KR); JEONG, Joong Hwan, Bucheon-si Gyeonggi-do 14500 (KR); KIM, Bong Yoon, Goyang-si Gyeonggi-do 10469 (KR)
(74) Representative: dompatent von Kreisler Selting Werner - Partnerschaft von Patent- und Rechtsanwälten mbB
(86) International application number: PCT/KR2021/012558
(87) International publication number: WO 2022/060069

(57) **Abstract**

Disclosed is a specimen collection stick including a support unit having a predetermined length and a specimen collection unit located at a front end of the support unit, the specimen collection unit being configured to collect a specimen in a body that comes into contact with the specimen collection unit, whereby the amount of a specimen that is collected from of a subject is increased and the amount of the collected specimen that is dissolved or dispersed in a reagent or a solution from the specimen collection stick is increased.

## Description

### [Technical Field]

The present invention relates to a specimen collection stick, and more particularly to a specimen collection stick capable of scraping a specimen, such as saliva in the oral cavity or the nasal cavity of a subject, thereby collecting the specimen, such as saliva in the oral cavity or the nasal cavity of the subject.

### [Background Art]

A microbiological examination is an examination for determining whether a specimen has been contaminated by disease-causing germs. That is, when it is thought that a cause of illness is a microorganism, the microbiological examination is performed in order to establish a method of diagnosing, treating, and preventing the same. The microbiological examination is performed to determine whether a subject has been contaminated by disease-causing germs, such as a colon bacillus, a typhoid bacillus, staphylococcus, and pseudomonas.

In general, a specimen is collected from the body of the subject in order to perform the microbiological examination. The collected specimen is mixed with a reagent or solution for examination, and then examination is performed to determine whether there are disease-causing germs.

In order to collect a specimen, such as saliva or a bodily fluid, from bodily tissue of the subject, a tool called a collection stick, a brush, or a swab is used. That is, the swab for specimen collection is introduced into the body of the subject, a specimen is adhered to the swab, and the swab is withdrawn, whereby the specimen is collected.

In the conventional swab for specimen collection, a circular collection unit is formed at an end of the swab, which is formed in the shape of a bar. The collection unit of the swab is provided with a fiber layer, which is formed by attaching small-sized microfibers to the collection unit. A specimen permeates the fiber layer formed on the collection unit of the conventional swab, and the collection unit of the swab is withdrawn from the subject, whereby the specimen is collected.

In the conventional swab for specimen collection used to collect the specimen, however, it is necessary for the swab to remain inserted in the oral cavity or the nasal cavity of the subject for a predetermined time or more such that saliva or the like permeates the fiber layer, whereby the subject may experience great discomfort. Above all, a part of the fiber layer included in the swab for specimen collection may be separated from the collection unit of the swab and may remain in the body of the subject. When foreign matter, such as the fiber layer, remains in the body of the subject, a medically serious problem may occur. In addition, the amount of the specimen that is collected using the conventional swab is not sufficient, whereby examination accuracy may be lowered.

Therefore, there is a need for technology capable of solving such problems.

### [Disclosure]

### [Technical Problem]

The present invention has been made in view of the above problems, and it is an object of the present invention to provide a specimen collection stick capable of increasing the amount of a specimen that is collected during specimen collection, shortening time necessary for specimen collection, and preventing a problem in which fibers constituting a fiber layer of the conventional swab are separated from the swab.

### [Technical Solution]

A specimen collection stick according to a first embodiment of the present invention to accomplish the above object includes a rod-shaped or bar-shaped support unit having a predetermined length and a specimen collection unit located at a front end of the support unit, the specimen collection unit being configured to collect a specimen in a body of a subject that comes into contact with the specimen collection unit, the specimen collection unit including a plurality of collection disks arranged in a longitudinal direction of the support unit.

The specimen collection unit may further include a hub coupled to the support unit such that a front portion of the support unit is inserted into the hub, the hub being configured to support the plurality of collection disks.

Each of the collection disks may be formed in a plate shape having at least one curved or flat side surface, and an edge of each of the collection disks may be circular or oval.

The plurality of collection disks may be coupled to or formed at the hub such that the hub extends through a central part of each of the plurality of collection disks, and a predetermined gap may be provided between neighboring ones of the collection disks such that the specimen can remain in the gap.

A coupling recess or coupling hole may be formed in the hub such that the front portion of the support unit is inserted into the coupling recess or coupling hole, whereby the front portion of the support unit is coupled to the hub.

The hub may be provided in an outer surface thereof with a support unit fastening recess or support unit fastening hole configured to communicate with the coupling recess or coupling hole of the hub, and a hub fastening projection or hub fastening protrusion coupled to the support unit fastening recess or support unit fastening hole by fitting as the front portion of the support unit is inserted into the coupling recess or coupling hole may be formed on an outer surface of the front portion of the support unit.

In at least some of the plurality of collection disks, neighboring ones of the collection disks may have different diameters or different long radii.

The specimen collection stick may further include a handle unit connected to a rear end of the support unit, the handle unit having a predetermined length such that the handle unit can be gripped by a hand or fingers of an examiner.

A segment groove may be provided between the handle unit and the support unit such that the handle unit and the support unit can be separated from each other.

A coupling projection or coupling protrusion may be formed at an outer circumferential surface of the support unit so as to protrude therefrom, or a lid fastening groove may be formed in the outer circumferential surface of the support unit, such that the support unit can be fixed to a lid of a specimen container configured to receive the specimen collection unit therein by coupling.

A specimen collection stick according to a second embodiment of the present invention to accomplish the above object includes a rod-shaped or bar-shaped support unit having a predetermined length and a specimen collection unit located at a front end of the support unit, the specimen collection unit being configured to collect a specimen in a body of a subject that comes into contact with the specimen collection unit, the specimen collection unit including a plurality of collection blades disposed in rotational symmetry or axial symmetry with respect to a central axis of the specimen collection unit in a longitudinal direction thereof.

The specimen collection unit may further include a hub coupled to the support unit such that a front portion of the support unit is inserted into the hub, the hub being configured to support the plurality of collection blades.

Each of the collection blades may be formed in a plate shape having at least one curved or flat side surface.

The length of an inner side of each of the collection blades abutting the hub by coupling may be equal to or less than the length of an outer side thereof.

The plurality of collection blades arranged at an outer circumferential surface of the hub on the same circumference may constitute a blade group, and a plurality of neighboring blade groups may be disposed on the outer circumferential surface of the hub such that each of the blade groups is spaced apart from a corresponding one of the blade groups neighboring thereto by a predetermined distance.

The specimen collection stick may further include a handle unit connected to a rear end of the support unit, the handle unit having a predetermined length such that the handle unit can be gripped by a hand or fingers of an examiner.

A segment groove may be provided between the handle unit and the support unit such that the handle unit and the support unit can be separated from each other.

A coupling projection or coupling protrusion may be formed at an outer circumferential surface of the support unit so as to protrude therefrom, or a lid fastening groove may be formed in the outer circumferential surface of the support unit, such that the support unit can be fixed to a lid of a specimen container configured to receive the specimen collection unit therein by coupling.

Each of the collection blades or the hub may be made of a flexible or elastic material.

A specimen collection stick according to another embodiment of the present invention to accomplish the above object includes a rod-shaped or bar-shaped support unit having a predetermined length and a specimen collection unit located at a front end of the support unit, the specimen collection unit being configured to collect a specimen in a body of a subject that comes into contact with the specimen collection unit, wherein the specimen collection unit includes a collection blade having a plate shape, the collection blade being configured to inhibit separation of the specimen that comes into contact with the specimen collection unit and a hub coupled to the support unit such that a front portion of the support unit is inserted into the hub, the hub being configured to support a plurality of collection blades disposed on an outer circumferential surface thereof, and the plurality of collection blades disposed on the outer circumferential surface of the hub is disposed asymmetrically with respect to a central axis of the hub or the support unit in a longitudinal direction thereof.

A specimen collection stick according to a third embodiment of the present invention to accomplish the above object includes a rod-shaped or bar-shaped support unit having a predetermined length and a specimen collection unit located at a front end of the support unit, the specimen collection unit being configured to collect a specimen in a body of a subject that comes into contact with the specimen collection unit, the specimen collection unit including a screw blade spirally formed along a central axis of the support unit in a longitudinal direction thereof.

The specimen collection unit may further include a hub coupled to the support unit such that a front portion of the support unit is inserted into the hub, the hub being configured to support the screw blade disposed on an outer circumferential surface thereof.

A plurality of collection recesses may be formed in an outer edge of the screw blade.

At least some of the plurality of collection recesses may be formed in a direction perpendicular to a central axis of the hub or the support unit in a longitudinal direction thereof.

At least some of the plurality of collection recesses may be formed in a direction in which neighboring collection recesses are parallel to each other within a predetermined error range.

At least a part of the screw blade may be divided into a plurality of screw blade parts at least partially spaced apart from each other.

The screw blade or the hub may be made of a flexible or elastic material.

The specimen collection stick may further include a handle unit connected to a rear end of the support unit, the handle unit having a predetermined length such that the handle unit can be gripped by a hand or fingers of an examiner.

A segment groove may be provided between the handle unit and the support unit such that the handle unit and the support unit can be separated from each other.

A coupling projection or coupling protrusion may be formed at an outer circumferential surface of the support unit so as to protrude therefrom, or a lid fastening groove may be formed in the outer circumferential surface of the support unit, such that the support unit can be fixed to a lid of a specimen container configured to receive the specimen collection unit therein by coupling.

### [Advantageous Effects]

A specimen collection stick according to the present invention has effects in that a flocking process, which is required to manufacture the conventional swab for specimen collection, is not necessary, whereby it is possible to reduce the manufacturing cost of the specimen collection stick, and in that there is no fiber layer in the specimen collection stick, whereby it is possible to prevent a problem in which the fiber layer is separated from the specimen collection stick and remains in the body of a subject, unlike the conventional swab for specimen collection, and therefore it is possible to improve examination safety.

Also, in the specimen collection stick according to the present invention, it is possible to increase the amount of the specimen that is collected from the body of the subject, and it is possible to reduce time necessary for specimen collection, since absorption time for which the specimen permeates the fiber layer is not necessary, unlike the conventional swab. Consequently, the specimen collection stick according to the present invention has effects in that it is possible to shorten time necessary for specimen collection, to reduce inconvenience of the subject during specimen collection, and to improve examination accuracy with an increase in amount of the specimen that is collected.

### [Description of Drawings]

FIG. 1 is a perspective view schematically showing a specimen collection stick according to a first embodiment of the present invention.
FIG. 2 is a side view schematically showing a specimen collection unit of the specimen collection stick according to the first embodiment of the present invention.
FIG. 3 is a side view schematically showing fastening between the specimen collection unit and a support unit of the specimen collection stick according to the first embodiment of the present invention.
FIG. 4 is a view schematically showing the shapes of the support unit and a modified example of the specimen collection unit of the specimen collection stick according to the first embodiment of the present invention.
FIG. 5 is a view schematically showing a modified example of coupling between the specimen collection unit and the support unit of the specimen collection stick according to the first embodiment of the present invention.
FIG. 6 is a side view schematically showing a specimen collection stick according to a second embodiment of the present invention.
FIG. 7 is a side view schematically showing a specimen collection unit of the specimen collection stick according to the second embodiment of the present invention.
FIG. 8 is a front view schematically showing the specimen collection stick according to the second embodiment of the present invention.
FIG. 9 is a perspective view schematically showing the specimen collection unit of the specimen collection stick according to the second embodiment of the present invention.
FIG. 10 is a front view schematically showing the specimen collection stick according to the second embodiment of the present invention.
FIG. 11 is a side view schematically showing the specimen collection stick according to the second embodiment of the present invention.
FIG. 12 is a perspective view schematically showing the specimen collection unit of the specimen collection stick according to the second embodiment of the present invention.
FIGs. 13 to 15 are conceptual views schematically showing a part of the specimen collection unit of the specimen collection stick according to the second embodiment of the present invention.
FIGs. 16 and 17 are conceptual views schematically showing planar development of the specimen collection unit of the specimen collection stick according to the second embodiment of the present invention.
FIG. 18 is an incised side sectional view schematically showing the specimen collection unit of the specimen collection stick according to the second embodiment of the present invention.
FIG. 19 is a side perspective view schematically showing a specimen collection stick according to a third embodiment of the present invention.
FIG. 20 is a side view schematically showing a specimen collection unit of the specimen collection stick according to the third embodiment of the present invention.
FIG. 21 is a perspective view schematically showing the specimen collection unit of the specimen collection stick according to the third embodiment of the present invention.
FIG. 22 is a front view schematically showing a modified example of the specimen collection unit of the specimen collection stick according to the third embodiment of the present invention.
FIG. 23 is a perspective view schematically showing the modified example of the specimen collection unit of the specimen collection stick according to the third embodiment of the present invention.
FIG. 24 is a front view schematically showing an applied example of the specimen collection unit of the specimen collection stick according to the third embodiment of the present invention.
FIG. 25 is a perspective view schematically showing the applied example of the specimen collection unit of the specimen collection stick according to the third embodiment of the present invention.
FIG. 26 is a front view schematically showing another modified example of the specimen collection unit of the specimen collection stick according to the third embodiment of the present invention.
FIG. 27 is a perspective view schematically showing the other modified example of the specimen collection unit of the specimen collection stick according to the third embodiment of the present invention.

### [Best Mode]

The present invention may be variously changed and may have several embodiments, and therefore specific embodiments will be described in detail while being illustrated in the drawings. However, the present invention is not limited to the specific embodiments, and it should be understood that the present invention includes all alterations, equivalents, and substitutions falling within the idea and technical scope of the present invention. The present embodiments are provided to more specifically describe the present invention to a person having ordinary skill in the art to which the present invention pertains. Consequently, the shape of each element in the drawings may be exaggerated for clearer description. In describing the present invention, a detailed description of related known technology will be omitted when the same may obscure the subject matter of the present invention.

Although the terms, such as "first" and "second," may be used to describe various elements, the elements must not be defined by the terms. The terms are used only for the purpose of distinguishing one element from another and describing the same so as to be understood.

The terms used in the present invention are used only to describe a specific embodiment, not to define the present invention. Singular forms include plural forms unless mentioned otherwise.

It should be understood that the terms "comprises," "has," etc. specify the presence of stated features, numbers, steps, operations, elements, components, or combinations thereof described in this specification, but do not preclude the presence or addition of one or more other features, numbers, steps, operations, elements, components, or combinations thereof.

Hereinafter, embodiments of the present invention will be described with reference to the accompanying drawings, and the present invention will be described in detail to the extent to which a person having ordinary skill in the art to which the present invention pertains can easily implement the present invention. However, the present invention may be implemented in various different forms and is not limited to the embodiments described herein. Similar parts may be denoted by the same reference numerals throughout the specification.

Hereinafter, embodiments of a specimen collection stick according to the present invention will be described with reference to the drawings.

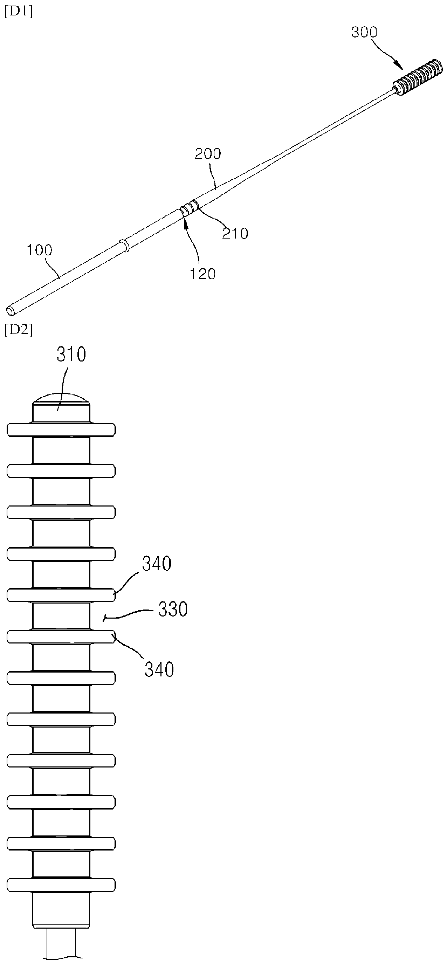

FIG. 1 is a perspective view schematically showing a specimen collection stick according to a first embodiment of the present invention, and FIG. 2 is a side view schematically showing a specimen collection unit of the specimen collection stick according to the first embodiment of the present invention.

Referring to FIGs. 1 and 2, the specimen collection stick according to the first embodiment of the present invention includes a support unit 200 and a specimen collection unit 300, and may further include a handle unit.

First, the support unit 200 is formed so as to have a shape, such as a rod or a bar, and has a predetermined length, as shown in the figures. The specimen collection unit 300 is coupled to a front end of the support unit 200. The support unit 200 supports the specimen collection unit 300. The handle unit is connected to the rear of the support unit 200. The support unit 200 supports the specimen collection unit 300 such that the specimen collection unit can be introduced into the body of a subject.

It is sufficient for the support unit 200 to support the specimen collection unit 300 such that the specimen collection unit can be introduced into the body of the subject in order to perform specimen collection, and the shape of the support unit is not limited to a specific shape.

Furthermore, it is preferable for the support unit 200 to be made of a polymer material that exhibits flexibility and elasticity such that the support unit can return to the original shape thereof while being flexibly deformed by force applied thereto.

Meanwhile, when the specimen collection unit 300 and the support unit 200 of the specimen collection stick 10 are withdrawn from a specimen container (not shown) using a tool, such as a pincette, it is necessary to wash the pincette in advance. In addition, after the support unit 200 of the specimen collection stick 10 is withdrawn from the specimen container using the pincette and the specimen collection unit 300 and the support unit 200 of the specimen collection stick 10 are located at a position intended by an examiner, it is necessary to wash the used pincette again.

Since it is necessary to wash the pincette whenever the pincette is used in the process of withdrawing the specimen collection stick from the specimen container, as described above, specimen examination efficiency is lowered. In order to withdraw the specimen collection unit 300 and the support unit 200 of the specimen collection stick 10 from the specimen container without using the pincette, therefore, the following construction may be provided.

That is, a coupling projection or coupling protrusion 210 may be formed at an outer surface of the support unit 200 so as to protrude from the outer surface thereof such that the coupling projection or coupling protrusion is fixed to a cover or lid of the specimen container by coupling.

The cover or lid (not shown) of the specimen container (not shown) may be provided at an inside thereof with a fixing groove or fitting projection (not shown) configured to allow the support unit 200 of the specimen collection stick to be fixed to the cover or lid (not shown) of the specimen container therethrough. The coupling projection or coupling protrusion 210 of the support unit may be coupled to the fixing groove or fitting projection provided at the inside of the cover or lid of the specimen container by engagement or fitting.

Since the support unit 200 can be fixed to the cover or lid of the specimen container, as described above, it is possible to easily withdraw the support unit 200 from the specimen container together with the cover or lid of the specimen container when the cover or lid is removed from the specimen container without using a tool, such as a pincette, which is preferable.

FIG. 1 shows a ring-shaped coupling projection or coupling protrusion 210 formed on the outer surface of the support unit 200 in a circumferential direction thereof as an illustrative example of the coupling projection or coupling protrusion 210. As shown in FIG. 1, it is also preferable for one or more ring-shaped coupling projections or coupling protrusions 210 to be provided on the outer surface of the support unit 200 at the rear of the support unit 200.

As long as the support unit 200 can be coupled to the cover of the specimen container, as described above, it is possible to improve convenience in use, which is preferable.

As previously described, the handle unit 100 is located at the rear of the support unit 200. That is, a rear end of the support unit 200 is connected to the handle unit 100.

The handle unit 100 is connected to the rear end of the support unit 200, and the handle unit has a predetermined length such that the handle unit can be gripped by the hand or fingers of the examiner.

As shown in the figures, the handle unit 100 is formed in the shape of a rod or a bar having a predetermined length. The handle unit 100 is gripped by the hand or fingers of the examiner. The examiner may perform control such that the specimen collection unit 300 is introduced or inserted into the body of the subject using the handle unit 100.

Consequently, the specimen collection unit 300 may be introduced or inserted into the body of the subject through the oral cavity or the nasal cavity of the subject under control of the examiner using the handle unit 100.

As shown in the figures, it is preferable for a segment groove 120, such as a segment joint, capable of distinguishing between the handle unit 100 and the support unit 200 to be provided between the handle unit 100 and the rear end of the support unit 200. That is, it is preferable for the segment groove 120 to be provided between the handle unit 100 and the support unit 200 such that the handle unit 100 and the support unit 200 can be separated from each other.

After the specimen collection unit 300 is withdrawn from the body of the subject according to the intention of the examiner, who is a user, the specimen collection unit 300 and the support unit 200 are received in the specimen container.

Since it is not necessary to receive the handle unit 100 in the specimen container at this time, it is preferable to separate the handle unit 100 from the support unit 200. As shown in the figures, therefore, it is preferable for the segment joint or segment groove 120 to be provided between the support unit 200 and the handle unit 100 such that the support unit 200 and the handle unit 100 can be separated from each other as needed.

After the support unit 200 and the handle unit 100 are separated from each other, the support unit 200 may be coupled to the cover or lid of the specimen container, as previously described. It is preferable for the coupling projection or coupling protrusion 210 to be formed at an outer circumferential surface of the support unit so as to protrude therefrom or for a lid fastening groove to be formed in the outer circumferential surface of the support unit such that the support unit can be fixed to the cover or lid of the specimen container, in which the specimen collection unit is received, by coupling.

The specimen collection unit 300 is a part that is inserted or introduced into the body of the subject so as to come into contact with a specimen. That is, the specimen collection unit is a part that collects the specimen.

The specimen collection unit 300 is located at the front end of the support unit 200 in order to gather or collect a specimen in the body of the subject that comes into contact with the specimen collection unit. The specimen collection unit 300 includes a plurality of collection disks 340 arranged in a longitudinal direction of the support unit 200.

The specimen collection unit 300 may further include a hub 310 coupled to the support unit 200 such that front portions of the support unit 200 are inserted into the hub, the hub being configured to support the plurality of collection disks 340.

Each of the collection disks 340 is formed in the shape of a plate having at least one curved or flat side surface. In addition, the edge of the collection disk 340 is circular or oval. The collection disk 340 may have a disk shape.

As shown in the figures, the plurality of collection disks is coupled to or formed at the hub 310 such that the hub extends through a central part of each of the plurality of collection disks 340.

It is preferable for a predetermined gap to be provided between neighboring ones of the collection disks such that the specimen can remain in the gap when the specimen collection unit 300 is withdrawn from the body of the subject.

Each of the plurality of collection disks 340 is flexible and elastic. Consequently, each of the plurality of collection disks 340 abuts bodily tissue in the body of the subject. The examiner swings the specimen collection unit 300 in the body of the subject using the handle unit 100 such that the collection disks 340 can scrape or sweep the bodily tissue in which the specimen is present while abutting the bodily tissue. As a result, the specimen is adhered to the surface of each of the collection disks 340 or is held in the gap between the neighboring ones of the collection disks 340.

That is, the specimen is adhered to the surface of each of the collection disks 340 or is held in the gap between the neighboring ones of the collection disks 340 in the state in which the specimen collection unit 300 is inserted in the body of the subject, whereby the specimen remains on the specimen collection unit 300 when the specimen collection unit 300 is withdrawn from the body of the subject. The gap 330 between the neighboring ones of the collection disks 340 may also be referred to as a collection space or a collection gap.

Also, in at least some of the plurality of collection disks 340, it is also preferable for neighboring ones of the collection disks 340 to have different diameters or different long radii. Of course, all of the plurality of collection disks 340 may also have the same diameter or the same long radius.

It is preferable for the collection disks 340 to be manufactured by molding such that each of the collection disks has a diameter or size set depending on the part of the body of the subject into which the collection disks are inserted, such as the nasal cavity, the oral cavity, the anus, or the cervix. The specimen collection unit 300 including the collection disks 340 and the hub 310 may be made of a polymer material that exhibits flexibility and elasticity or an elastomer.

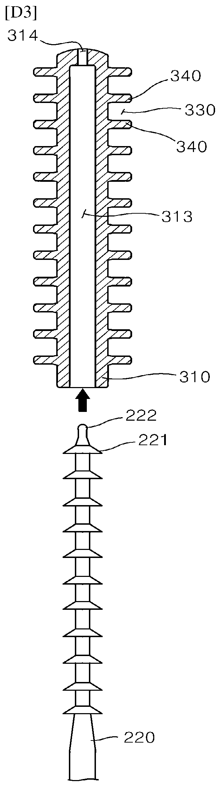

A further description will be given with reference to FIG. 3.

FIG. 3 is a side view schematically showing fastening between the specimen collection unit and the support unit of the specimen collection stick according to the first embodiment of the present invention.

Further referring to FIG. 3, it is preferable for a coupling recess or coupling hole 313 to be formed in the hub 310 such that the front portions 220, 221, and 222 of the support unit 200 are inserted into the coupling recess or coupling hole, whereby the front portions of the support unit 200 are coupled to the hub 310.

As shown in FIG. 3, it is preferable for a hub fastening projection or hub fastening protrusion 221 to be formed on the outer surface of the front of the support unit 200 as one of the front portions of the support unit 200.

When the support unit 200 is provided with the hub fastening projection or hub fastening protrusion 221, the specimen collection unit 300, which is configured to be coupled to the front end of the support unit 200, may be fastened to the front end of the support unit 200 so as not to be separated therefrom.

The hub 310 may be provided in a front end thereof with an air vent hole 314 configured to reduce the pressure of air in the coupling recess or coupling hole 313 of the hub 310 when the front portions 220, 221, and 222 of the support unit are inserted into the coupling recess or coupling hole. If the air vent hole 314 is not provided, the coupling recess or coupling hole 313 of the hub 310 is hermetically sealed by the front portions 220, 221, and 222 of the support unit 200 inserted into the coupling recess or coupling hole, whereby the pressure of air in the coupling recess or coupling hole 313 of the hub 310 is increased, and therefore fastening between the hub 310 and the support unit 200 is disturbed. When the air vent hole 314 is provided in the front end of the hub 310, therefore, air in the coupling recess or coupling hole 313 of the hub 310 is exhausted through the air vent hole 314 when the hub 310 and the support unit 200 are fastened to each other, which assists in fastening between the hub 310 and the support unit 200.

It is preferable for the inner diameter of the air vent hole 314 to be equal to or less than the outer diameter of the front end 222 of the support unit. Furthermore, it is also preferable for the front end 222 of the support unit to have an outer diameter gradually reduced outwards such that the front end of the support unit is tapered, as shown in the figures.

When the hub 310 and the support unit 200 are fastened to each other, the front end 222 of the support unit is inserted into the air vent hole 314, whereby the air vent hole 314 is blocked, and therefore the introduction of outside air through the air vent hole 314 is prevented.

As a result, the pressure in a space between the coupling recess or coupling hole 313 of the hub 310 and the front portions 220, 221, and 222 of the support unit 200 is lower than outside pressure, which assists in inhibiting separation of the hub 310 from the support unit 200.

Furthermore, it is preferable for the front portion 220 of the support unit 200 to be tapered, as shown in the figures, such that the coupling recess or coupling hole 313 of the hub 310 is hermetically sealed.

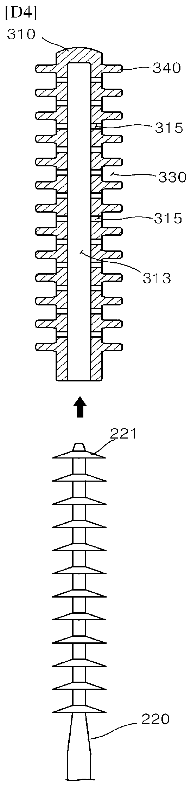

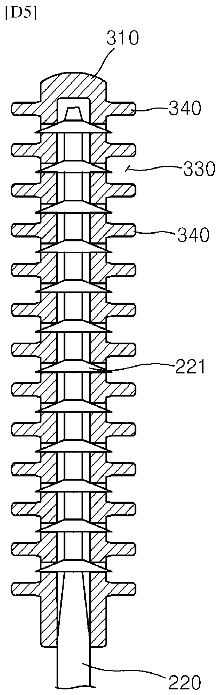

Meanwhile, the support unit and a modified example of the specimen collection unit may be fastened to each other, as shown in FIG. 4 or 5, which shows a modified example of FIG. 3.

A description will be given with reference to FIGs. 4 and 5.

FIG. 4 is a view schematically showing the shapes of the support unit and a modified example of the specimen collection unit of the specimen collection stick according to the embodiment of the present invention, and FIG. 5 is a view schematically showing a modified example of coupling between the specimen collection unit and the support unit of the specimen collection stick according to the embodiment of the present invention.

As shown in FIGs. 4 and 5, the hub 310 of the specimen collection unit 300 is provided in the outer surface thereof with a support unit fastening recess or support unit fastening hole 315 configured to communicate with the coupling recess or coupling hole 313 of the hub 310. Of course, as previously described, the plurality of collection disks 340 is also provided, and specimen collection using the plurality of collection disks 340 is identical to the above description.

It is preferable for the hub fastening projection or hub fastening protrusion 221, which is coupled to the support unit fastening recess or support unit fastening hole 315 by fitting as the front portions 220 and 221 of the support unit 200 are inserted into the coupling recess or coupling hole 313, to be formed on outer surfaces of the front portions of the support unit 200.

Since the hub fastening projection or hub fastening protrusion 221 of the support unit 200 is fastened to the support unit fastening recess or support unit fastening hole 315 formed in the hub 310 by insertion, as schematically shown in FIG. 5, the hub 310 and the support unit 200 are fastened to each other so as not to be separated from each other.

In the specimen collection stick according to the first embodiment of the present invention, as described above, the amount of the specimen that is collected by the plurality of collection disks included in the specimen collection unit and in the gap between the collection disks is greater than the amount of the specimen collected by the conventional swab for specimen collection.

As described above, it is possible to increase the amount of the specimen that is collected from the subject and to increase the amount of the collected specimen that is dissolved or dispersed in a reagent or a solution from the specimen collection stick.

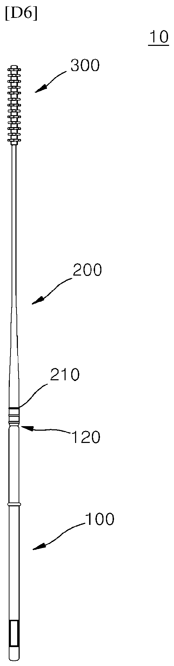

Next, a specimen collection stick according to a second embodiment of the present invention will be described with reference to FIGs. 6 to 9.

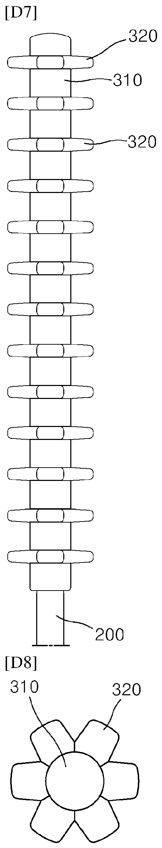

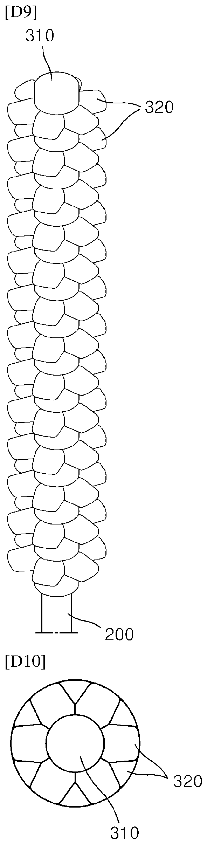

FIG. 6 is a side view schematically showing a specimen collection stick according to a second embodiment of the present invention, FIG. 7 is a side view schematically showing a specimen collection unit of the specimen collection stick according to the second embodiment of the present invention, FIG. 8 is a front view schematically showing the specimen collection stick according to the second embodiment of the present invention, and FIG. 9 is a perspective view schematically showing the specimen collection unit of the specimen collection stick according to the second embodiment of the present invention.

Referring to FIGs. 6 to 9, the specimen collection stick 10 according to the second embodiment of the present invention includes a support unit 200 and a specimen collection unit 300, and may further include a handle unit 100.

First, the support unit 200 is formed so as to have a shape, such as a rod or a bar, and has a predetermined length, as shown in the figures. The specimen collection unit 300 is located at the front of the support unit 200, and the support unit 200 supports the specimen collection unit 300. The handle unit 100 is connected to the rear of the support unit 200. The support unit 200 supports the specimen collection unit 300 such that the specimen collection unit can be introduced into the body of a subject.

The front of the support unit 200 and the specimen collection unit 300 may be made of a single material, such as a polymer material or an elastomer material. Alternatively, the support unit 200 and the specimen collection unit 300 may be made of different materials, and the support unit 200 and the specimen collection unit 300 may be coupled to each other. The specimen collection unit 300 may be formed at the front of the support unit 200 by fusion.

It is sufficient for the support unit 200 to support the specimen collection unit 300 such that the specimen collection unit can be introduced into the body of the subject in order to perform specimen collection, and the shape of the support unit is not limited to a specific shape.

Furthermore, it is preferable for the support unit 200 to be made of a polymer material that exhibits flexibility and elasticity such that the support unit can return to the original shape thereof while being flexibly deformed by force applied thereto.

Meanwhile, when the specimen collection unit 300 and the support unit 200 of the specimen collection stick 10 is withdrawn from a specimen container (not shown) using a tool, such as a pincette, it is necessary to wash the pincette in advance. In addition, after the support unit 200 of the specimen collection stick 10 is withdrawn from the specimen container using the pincette and the specimen collection unit 300 and the support unit 200 of the specimen collection stick 10 are located at a position intended by an examiner, it is necessary to wash the used pincette again.

Since it is necessary to wash the pincette whenever the pincette is used in the process of withdrawing the specimen collection stick 10 from the specimen container, as described above, specimen examination efficiency is lowered. In order to withdraw the specimen collection unit 300 and the support unit 200 of the specimen collection stick 10 from the specimen container without using the pincette, therefore, the following construction may be provided.

That is, a coupling projection or coupling protrusion 210 may be formed at an outer surface of the support unit 200 so as to protrude from the outer surface thereof such that the coupling projection or coupling protrusion is fixed to a cover or lid of the specimen container by coupling.

The cover or lid (not shown) of the specimen container (not shown) may be provided at an inside thereof with a fixing groove or fitting projection (not shown) configured to allow the support unit 200 of the specimen collection stick to be fixed to the cover of the specimen container therethrough. The coupling projection or coupling protrusion 210 of the support unit may be coupled to the fixing groove or fitting projection provided at the inside of the cover of the specimen container by engagement or fitting.

Since the support unit 200 can be fixed to the cover or lid of the specimen container, as described above, it is possible to easily withdraw the support unit 200 from the specimen container together with the cover or lid of the specimen container when the cover or lid is removed from the specimen container without using a tool, such as a pincette, which is preferable.

FIG. 6 shows a ring-shaped coupling projection or coupling protrusion 210 formed on the outer surface of the support unit 200 in a circumferential direction thereof as an illustrative example of the coupling projection or coupling protrusion 210. As shown in FIG. 6, it is also preferable for one or more ring-shaped coupling projections or coupling protrusions to be provided on the outer surface of the support unit 200.

As long as the support unit 200 can be coupled to the cover of the specimen container, as described above, it is possible to improve convenience in use, which is preferable.

As previously described, the handle unit 100 is located at the rear of the support unit 200. That is, a rear end of the support unit 200 is connected to the handle unit 100.

The handle unit 100 is connected to the rear end of the support unit 200, and the handle unit has a predetermined length such that the handle unit can be gripped by the hand or fingers of the examiner.

As shown in the figures, the handle unit 100 is formed in the shape of a rod or a bar having a predetermined length. The handle unit 100 is gripped by the hand or fingers of the examiner. The examiner may perform control such that the specimen collection unit 300 is introduced or inserted into the body of the subject using the handle unit 100.

Consequently, the specimen collection unit 300 may be introduced or inserted into the body of the subject through the oral cavity or the nasal cavity of the subject under control of the examiner using the handle unit 100.

As shown in the figures, it is preferable for a segment groove 120, such as a segment joint, capable of distinguishing between the handle unit 100 and the support unit 200 to be provided between the handle unit 100 and the rear end of the support unit 200. That is, it is preferable for the segment groove 120 to be provided between the handle unit 100 and the support unit 200 such that the handle unit 100 and the support unit 200 can be separated from each other.

After the specimen collection unit 300 is withdrawn from the body of the subject according to the intention of the examiner, who is a user, the specimen collection unit 300 and the support unit 200 are received in the specimen container.

Since it is not necessary to receive the handle unit 100 in the specimen container at this time, it is preferable to separate the handle unit 100 from the support unit 200. As shown in the figures, therefore, it is preferable for the segment joint or segment groove 120 to be provided between the support unit 200 and the handle unit 100 such that the support unit 200 and the handle unit 100 can be separated from each other as needed.

After the support unit 200 and the handle unit 100 are separated from each other, the support unit 200 may be coupled to the cover of the specimen container, as previously described. It is preferable for the coupling projection or coupling protrusion 210 to be formed at an outer circumferential surface of the support unit so as to protrude therefrom or for a lid fastening groove to be formed in the outer circumferential surface of the support unit such that the support unit can be fixed to the cover or lid of the specimen container, in which the specimen collection unit is received, by coupling.

As shown in FIGs. 7 to 9 in more detail, the specimen collection unit 300 is a part that is inserted or introduced into the body of the subject so as to come into contact with a specimen. That is, the specimen collection unit is a part that collects the specimen.

The specimen collection unit 300 is located at a front end of the support unit 200 in order to collect a specimen in the body of the subject that comes into contact with the specimen collection unit. The specimen collection unit 300 includes a plurality of collection blades 320 arranged or disposed in rotational symmetry or axial symmetry with respect to a central axis of the specimen collection unit in a longitudinal direction thereof.

That is, the specimen collection unit 300 includes at least one collection blade 320 configured to allow a specimen brought into contact therewith to be adhered thereto or to be held thereby and to be withdrawn from the body of the subject in that state.

The specimen collection unit may further include a hub 310 coupled to the support unit 200 at the front of the support unit 200, the hub being configured to support the plurality of collection blades 320 on an outer surface thereof. That is, the hub 310 is coupled to or formed at the support unit 200 such that a front portion of the support unit 200 is inserted into the hub, and supports the plurality of collection blades 320.

The plurality of collection blades 320 of the specimen collection unit 300 may also be directly connected to the front end of the support unit 200.

In addition, as shown in FIGs. 7 to 9, a part of each of the plurality of collection blades 320 is formed at the hub 310 so as to be coupled thereto. The plurality of collection blades 320 thus formed is supported by the hub 310. That is, a part of each of the collection blades 320 is formed at the outer surface of the hub 310 so as to be coupled thereto such that the collection blades are supported by the hub 310. As described above, the collection blades 320 are provided at the outer surface of the hub 310.

It is preferable for each of the collection blades 320 or the hub 310 to be made of a material that exhibits flexibility or elasticity. That is, each of the collection blades or the hub may be made of a polymer material that exhibits flexibility and elasticity or an elastomer material.

The hub 310 configured to support the collection blades 320 is coupled to a part of the front of the support unit 200. The hub 310 is supported by the support unit 200. It is preferable for a central axis of the hub 310 in a longitudinal direction thereof to be aligned with a central axis of the support unit 200 in a longitudinal direction thereof.

Each of the collection blades 320 is formed so as to protrude from the central axis of the hub 310 or the support unit 200 in an outward direction, i.e. a centrifugal direction, by a predetermined size such that the collection blade can come into contact with a specimen.

It is preferable for each of the collection blades 320 to be formed in the shape of a plate having at least one curved or flat side surface. Also, it is preferable for outer corners of each collection blade having a fan shape or a plate shape to be rounded or curved.

When the outer corners of each collection blade having a fan shape or a plate shape are rounded or curved, it is possible to inhibit a possibility of a wound occurring in the body of the subject during the collection process, which is preferable.

The length of an inner side of each collection blade 320 abutting the hub 310 by coupling may be equal to or less than the length of an outer side thereof.

Since the hub 310 and the plurality of collection blades 320 are provided, as described above, the surface area of the specimen collection unit 300 is greater than the surface area of the conventional swab, as shown in the figures.

Each of the plurality of collection blades 320 is flexible and elastic. Consequently, each of the plurality of collection blades 320 abuts bodily tissue in the body of the subject. The examiner swings the specimen collection unit 300 in the body of the subject using the handle unit 100 such that the collection blades 320 can scrape or sweep the bodily tissue in which the specimen is present while abutting the bodily tissue. As a result, the specimen is adhered to the surface of each of the collection blades 320 or is held in a gap between neighboring ones of the collection blades 320.

That is, the specimen is adhered to the surface of each of the collection blades 320 or is held in the gap between the neighboring ones of the collection blades 320 in the state in which the specimen collection unit 300 is inserted in the body of the subject, whereby the specimen remains on the specimen collection unit 300 when the specimen collection unit 300 is withdrawn from the body of the subject. The gap between the neighboring ones of the collection blades 320 may also be referred to as a collection space or a collection gap.

The longitudinal section of the hub 310 in the central axis direction thereof may be circular. Of course, the longitudinal section of the hub may be quadrangular or polygonal. In the figures, the longitudinal section of the hub 310 is shown as being circular by way of example.

It is preferable for the collection blades 320 to be manufactured by molding such that each of the collection blades has a size set depending on the part of the body of the subject into which the collection disks are inserted, such as the nasal cavity, the oral cavity, the anus, or the cervix. The specimen collection unit 300 including the collection blades 320 and the hub 310 may be made of a polymer material that exhibits flexibility and elasticity or an elastomer.

As shown in the figures, the plurality of collection blades 320 is disposed or arranged at an outer circumferential surface of the hub 310 on the same circumference. FIG. 8 shows an example in which six collection blades 320 are disposed on the same circumference; however, the number of the collection blades 320 is not particularly restricted.

The plurality of collection blades 320 disposed or arranged at the outer circumferential surface of the hub 310 on the same circumference may constitute a blade crew or a blade group.

As shown in FIG. 7 or 9, a plurality of blade groups is disposed so as to be spaced apart from each other in the central axis direction of the hub 310.

The plurality of blade groups may be sequentially arranged along the central axis of the hub 310 in the longitudinal direction thereof.

It is preferable for the plurality of neighboring blade groups to be disposed on the outer circumferential surface of the hub 310 such that each of the blade groups is spaced apart from a corresponding one of the blade groups neighboring thereto by a predetermined distance. Of course, it is also preferable for the distance between the blade groups to be uniform.

As shown in FIG. 7, the neighboring blade groups are spaced apart from each other. A specimen entering a space between the blade groups spaced apart from each other comes into contact with or is adhered to the surface of each of the collection blades 320 or the surface of the hub 310. As a result, it is possible to achieve an effect in that the specimen is held by the surfaces of the collection blades 320 and the surface of the hub 310 in the vicinity of the specimen.

In addition, as shown in FIGs. 10 to 12, an applied example may be provided.

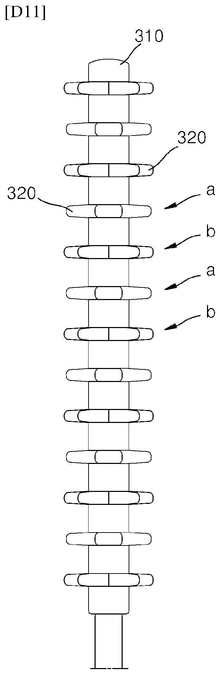

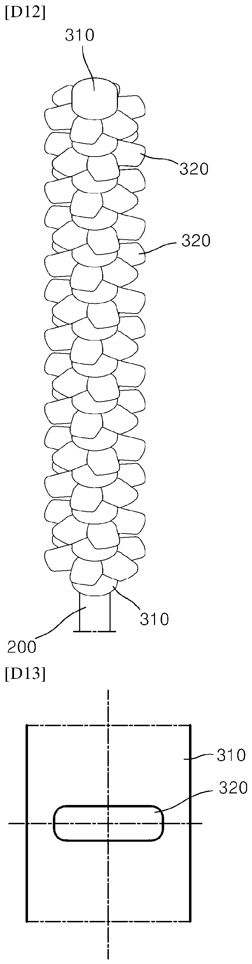

FIG. 10 is a front view schematically showing the specimen collection stick according to the second embodiment of the present invention, FIG. 11 is a side view schematically showing the specimen collection stick according to the second embodiment of the present invention, and FIG. 12 is a perspective view schematically showing the specimen collection unit of the specimen collection stick according to the second embodiment of the present invention.

As shown in FIGs. 10 to 12, the disposition of the collection blades 320 in one blade group is different from the disposition of the collection blades 320 in another blade group neighboring thereto.

That is, as shown in FIG. 9, each of the collection blades 320 in one blade group is aligned with a corresponding one of the collection blades 320 in another blade group neighboring thereto so as to form a line. In FIGs. 10 to 12, however, the collection blades 320 are not aligned with each other but are rotated by a predetermined angle about the central axis of the hub 310 in the longitudinal direction thereof.

As described above, the positions at which the collection blades 320 are formed in the blade groups may be different from each other, which is preferable.

As shown in FIG. 11, the collection blades 320 in the blade group indicated by reference symbol a and the collection blades 320 in the blade group indicated by reference symbol b are rotated by a predetermined angle about the central axis of the hub 310 in the longitudinal direction thereof so as to be aligned with each other in axial symmetry or rotational symmetry.

In addition, as shown in FIGs. 11 and 12, the blade group indicated by reference symbol a and the blade group indicated by reference symbol b are alternately disposed. Since the blade groups, in which the pluralities of collection blades 320 are coupled to the hub 310 in different directions, are alternately disposed in an axial direction of the hub 320, as described above, it is possible to improve specimen collection efficiency, which is preferable.

Next, the structure in which the collection blade is coupled to the hub will be described with reference to FIGs. 13 to 15.

FIGs. 13 to 15 are conceptual views schematically showing a part of the specimen collection unit of the specimen collection stick according to the embodiment of the present invention, i.e. the structure in which the collection blade 320 is coupled to the hub 310.

For convenience of understanding and description, only one collection blade 320 is shown as being coupled to the hub 310 in FIGs. 13 to 15.

As shown in FIG. 13, the collection blade 320 may be coupled to the hub 310 such that a central axis of the collection blade in a horizontal direction thereof is perpendicular to the central axis of the hub in the longitudinal direction thereof.

In addition, the central axes of the plurality of collection blades in the horizontal direction thereof may be inclined relative to the central axis of the hub in the longitudinal direction thereof by a predetermined angle, which is preferable.

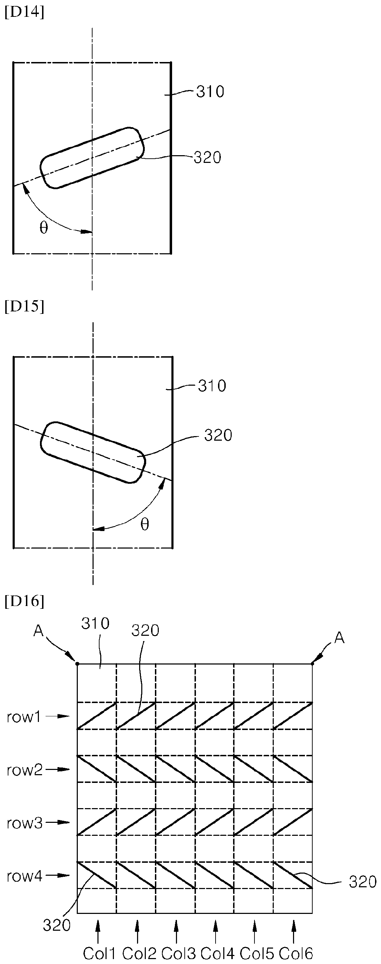

As shown in FIG. 14, the collection blade 320 may be formed at the surface of the hub 310 such that the central axis of the collection blade 320 in the horizontal direction thereof is inclined relative to the central axis of the hub 310 in the longitudinal direction thereof upwards in a rightward direction by a predetermined angle θ, which is also preferable. As shown in FIG. 15, the collection blade 320 may be formed at the surface of the hub 310 such that the central axis of the collection blade 320 in the horizontal direction thereof is inclined relative to the central axis of the hub 310 in the longitudinal direction thereof downwards in the rightward direction by a predetermined angle θ, which is also preferable.

As described above, the plurality of collection blades 320 may be coupled to the hub 310 such that a horizontal axis or horizontal plane of each of collection blades is inclined relative to the central axis of the hub in the longitudinal direction thereof by a predetermined angle, which is also preferable.

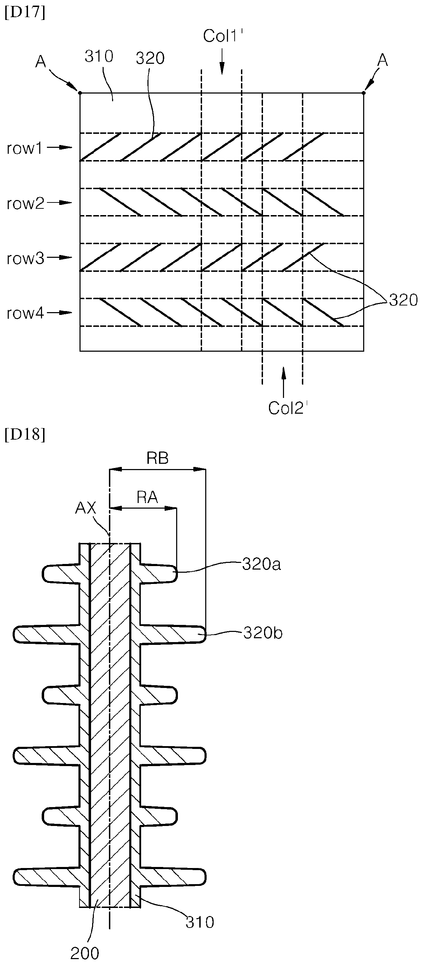

FIGs. 16 and 17 are conceptual views schematically showing planar development of the specimen collection unit of the specimen collection stick according to the embodiment of the present invention.

For convenience of understanding and description, an illustrative example in which six collection blades 320 are disposed on the same row to constitute one blade group and four blade group are disposed is schematically shown, wherein the planar shape of the specimen collection unit when the outer surface of the hub 310, the longitudinal section of which is circular, is developed in a planar shape is schematically shown.

Here, reference symbol A indicates a predetermined point on the surface of the hub 310, and when the hub is bent such that a left point A and a right point A of FIG. 16 come into contact with each other, the shape of the hub is formed as previously described.

First, as shown in FIG. 16, six collection blades 320 belonging to a first row row1 constitute one blade group. In addition, six collection blades 320 belonging to each of a second row row2, a third row row3, and a fourth row row4 constitute one blade group.

Each of the six collection blades 320 belonging to the first row row1 and the six collection blades 320 belonging to the third row row3 is coupled to the hub 310 such that each collection blade 320 is inclined relative to the central axis of the hub upwards in the rightward direction by a predetermined angle, as previously described with reference to FIG. 14.

In addition, each of the six collection blades 320 belonging to the second row row2 and the six collection blades 320 belonging to the fourth row row4 is coupled to the hub 310 such that each collection blade 320 is inclined relative to the central axis of the hub downwards in the rightward direction by a predetermined angle, as previously described with reference to FIG. 15.

As described above, the collection blades 320 may be disposed on the surface of the hub 310 such that the direction in which the collection blades 320 belonging to one blade group are inclined and the direction in which the collection blades 320 belonging to another blade group neighboring thereto are inclined are different from each other, as shown in FIG. 16, which is preferable.

As described above, it is also preferable for the inclination angle of the collection blades 320 belonging to one blade group and the inclination angle of the collection blades 320 belonging to another blade group neighboring thereto to be different from each other.

In addition, as shown in FIG. 16, each of the collection blades 320 commonly belongs to one column. That is, the collection blades 320 are disposed in a state of being aligned in the longitudinal direction of the central axis of the hub 310.

As shown in FIG. 17, which shows an applied example of FIG. 16, each of the six collection blades 320 belonging to the first row row1 and the six collection blades 320 belonging to the third row row3 is coupled to the hub 310 such that each collection blade 320 is inclined relative to the central axis of the hub upwards in the rightward direction by a predetermined angle, as previously described with reference to FIG. 14.

In addition, one of the collection blades 320 belonging to the first row row1 and one of the collection blades 320 belonging to the third row row3 are aligned with each other on one column, such as a column indicated by reference symbol Coll'.

In addition, each of the six collection blades 320 belonging to the second row row2 and the six collection blades 320 belonging to the fourth row row4 is coupled to the hub 310 such that each collection blade 320 is inclined relative to the central axis of the hub downwards in the rightward direction by a predetermined angle, as previously described with reference to FIG. 15. Here, one of the collection blades 320 belonging to the second row row2 and one of the collection blades 320 belonging to the fourth row row4 are aligned with each other in one column, such as a column indicated by reference symbol Col2'.

In addition, the collection blades 320 belonging to the first row row1 and the collection blades 320 belonging to the second row row2 are not aligned with each other. This arrangement of the collection blades 320 is possible and preferable.

FIG. 18 is an incised side sectional view schematically showing the specimen collection unit of the specimen collection stick according to the embodiment of the present invention.

As shown in FIG. 18, it is also preferable for a plurality of collection blades 320a and 320b disposed so as to neighbor each other in the longitudinal direction of the central axis of the hub 310 to have different lengths RA and RB from a central axis AX of the hub in an outward direction of each of the collection blades 320a and 320b, i.e. a centrifugal direction thereof.

Furthermore, as shown in FIG. 18, it is also preferable for the plurality of collection blades 320 to be disposed in the central axis direction of the hub 310 such that the different lengths from the central axis AX of the hub in the centrifugal direction of each of the collection blades 320 alternately appear.

Meanwhile, a modified embodiment of the specimen collection stick may be provided as follows.

The specimen collection stick may include a rod-shaped or bar-shaped support unit having a predetermined length and a specimen collection unit located at a front end of the support unit, the specimen collection unit being configured to collect a specimen in the body of a subject that comes into contact with the specimen collection unit, wherein the specimen collection unit may include a collection blade having a plate shape, the collection blade being configured to inhibit separation of the specimen that comes into contact with the specimen collection unit, and a hub coupled to the support unit such that a front portion of the support unit is inserted into the hub, the hub being configured to support a plurality of collection blades disposed on an outer circumferential surface thereof, and wherein the plurality of collection blades disposed on the outer circumferential surface of the hub may be disposed asymmetrically with respect to a central axis of the hub or the support unit in a longitudinal direction thereof. More specifically, the plurality of collection blades disposed on the outer circumferential surface of the hub may be disposed in asymmetry on a circumference having the central axis of the hub or the support unit in the longitudinal direction thereof as the center.

Here, the support unit and the hub of the specimen collection unit are identical to those previously described; however, the plurality of collection blades is modified in that the plurality of collection blades is disposed in asymmetry on a circumference having the central axis of the hub or the support unit in the longitudinal direction thereof as the center.

For example, the collection blades may be disposed such that the number of left collection blades and the number of right collection blades are different from each other based on the central axis of the hub. As a concrete example, three collection blades may be disposed at the left and two or four collection blades may be disposed at the right based on the central axis of the hub.

In addition, some of the collection blades may be different in size from the remainder of the collection blades based on the central axis of the hub.

As a concrete example, the size of some of the collection blades disposed so as to surround the central axis of the hub may be less than the size of the remainder of the collection blades. In an applied example, collection blades having different sizes may be alternately disposed so as to surround the central axis of the hub.

Since the plurality of collection blades is disposed asymmetrically with respect to the central axis of the hub or the support unit in the longitudinal direction thereof, as described above, it is possible to increase the amount of the specimen that is collected and remains, which is preferable.

In the specimen collection stick according to the second embodiment of the present invention, as described above, the amount of the specimen that is collected by the plurality of collection blades 320 included in the specimen collection unit 300 is greater than the amount of the specimen collected by the conventional swab for specimen collection.

Consequently, it is possible to increase the amount of the specimen that is collected from the subject and to increase the amount of the collected specimen that is dissolved or dispersed in a reagent or a solution from the specimen collection stick.

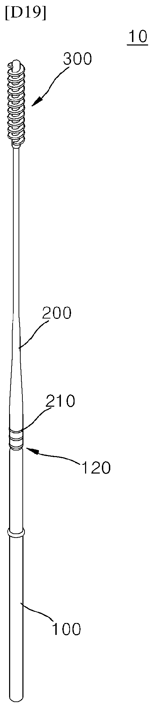

Next, a specimen collection stick according to a third embodiment of the present invention will be described with reference to FIGs. 19 to 25.

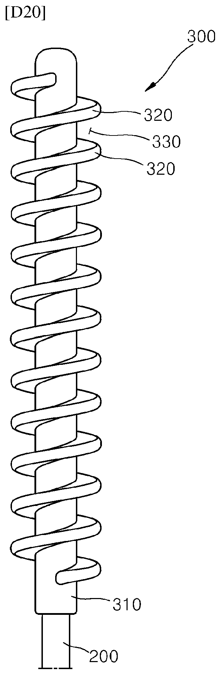

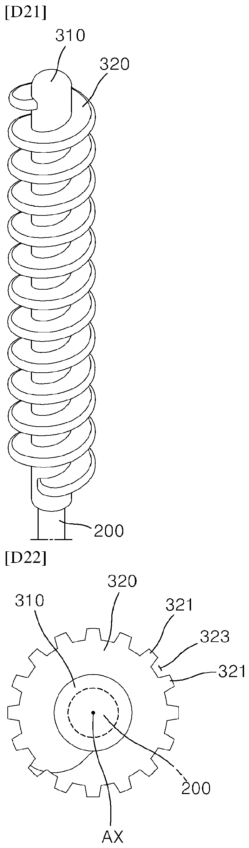

First, FIG. 19 is a side perspective view schematically showing a specimen collection stick according to a third embodiment of the present invention, FIG. 20 is a side view schematically showing a specimen collection unit of the specimen collection stick according to the third embodiment of the present invention, and FIG. 21 is a perspective view schematically showing the specimen collection unit of the specimen collection stick according to the third embodiment of the present invention.

Referring to FIGs. 19 to 21, the specimen collection stick 10 according to the third embodiment of the present invention includes a support unit 200 and a specimen collection unit 300, and may further include a handle unit 100.

First, the support unit 200 is formed so as to have a shape, such as a rod or a bar, and has a predetermined length. The specimen collection unit 300 is located at the front of the support unit 200, and supports the specimen collection unit 300. The handle unit 100 is connected to the rear of the support unit 200. The support unit 200 supports the specimen collection unit 300 such that the specimen collection unit can be introduced into the body of a subject.

The front of the support unit 200 and the specimen collection unit 300 may be made of a single material, such as a polymer material or an elastomer material. Alternatively, the support unit 200 and the specimen collection unit 300 may be made of different materials, and the support unit 200 and the specimen collection unit 300 may be coupled to each other. The specimen collection unit 300 may be formed at the front of the support unit 200 by fusion.

It is sufficient for the support unit 200 to support the specimen collection unit 300 such that the specimen collection unit can be introduced into the body of the subject in order to perform specimen collection, and the shape of the support unit is not limited to a specific shape.

Furthermore, it is preferable for the support unit 200 to be made of a polymer material that exhibits flexibility and elasticity such that the support unit can return to the original shape thereof while being flexibly deformed by force applied thereto.

Meanwhile, when the specimen collection unit 300 and the support unit 200 of the specimen collection stick 10 are withdrawn from a specimen container (not shown) using a tool, such as a pincette, it is necessary to wash the pincette in advance. In addition, after the support unit 200 of the specimen collection stick 10 is withdrawn from the specimen container using the pincette and the specimen collection unit 300 and the support unit 200 of the specimen collection stick 10 are located at a position intended by an examiner, it is necessary to wash the used pincette again.

Since it is necessary to wash the pincette whenever the pincette is used in the process of withdrawing the specimen collection stick 10 from the specimen container, as described above, specimen examination efficiency is lowered. In order to withdraw the specimen collection unit 300 and the support unit 200 of the specimen collection stick 10 from the specimen container without using the pincette, therefore, the following construction may be provided.

That is, a coupling projection or coupling protrusion 210 may be formed at an outer surface of the support unit 200 so as to protrude from the outer surface thereof such that the coupling projection or coupling protrusion is fixed to a cover or lid of the specimen container by coupling.

The cover or lid (not shown) of the specimen container (not shown) may be provided at an inside thereof with a fixing groove or fitting projection (not shown) configured to allow the support unit 200 of the specimen collection stick to be fixed to the cover of the specimen container therethrough. The coupling projection or coupling protrusion 210 of the support unit may be coupled to the fixing groove or fitting projection provided at the inside of the cover of the specimen container by engagement or fitting.

Since the support unit 200 can be fixed to the cover or lid of the specimen container, as described above, it is possible to easily withdraw the support unit 200 from the specimen container together with the cover or lid of the specimen container when the cover or lid is removed from the specimen container without using a tool, such as a pincette, which is preferable.

FIG. 19 shows a ring-shaped coupling projection or coupling protrusion 210 formed on the outer surface of the support unit 200 in a circumferential direction thereof as an illustrative example of the coupling projection or coupling protrusion 210. As shown in FIG. 19, it is also preferable for one or more ring-shaped coupling projections or coupling protrusions to be provided on the outer surface of the support unit 200.

As long as the support unit 200 can be coupled to the cover of the specimen container, as described above, it is possible to improve convenience in use, which is preferable.

As previously described, the handle unit 100 is located at the rear of the support unit 200. That is, a rear end of the support unit 200 is connected to the handle unit 100.

The handle unit 100 is connected to the rear end of the support unit 200, and the handle unit has a predetermined length such that the handle unit can be gripped by the hand or fingers of the examiner.

As shown in the figures, the handle unit 100 is formed in the shape of a rod or a bar having a predetermined length. The handle unit 100 is gripped by the hand or fingers of the examiner. The examiner may perform control such that the specimen collection unit 300 is penetrated or inserted into the body of the subject using the handle unit 100.

Consequently, the specimen collection unit 300 may be introduced or inserted into the body of the subject through the oral cavity or the nasal cavity of the subject under control of the examiner using the handle unit 100.

As shown in the figures, it is preferable for a segment groove 120, such as a segment joint, capable of distinguishing between the handle unit 100 and the support unit 200 to be provided between the handle unit 100 and the rear end of the support unit 200. That is, it is preferable for the segment groove 120 to be provided between the handle unit 100 and the support unit 200 such that the handle unit and the support unit can be separated from each other.

After the specimen collection unit 300 is withdrawn from the body of the subject according to the intention of the examiner, who is a user, the specimen collection unit 300 and the support unit 200 are received in the specimen container.

Since it is not necessary to receive the handle unit 100 in the specimen container at this time, it is preferable to separate the handle unit 100 from the support unit 200. As shown in the figures, therefore, it is preferable for the segment joint or segment groove 120 to be provided between the support unit 200 and the handle unit 100 such that the support unit 200 and the handle unit 100 can be separated from each other as needed.

After the support unit 200 and the handle unit 100 are separated from each other, the support unit 200 may be coupled to the cover of the specimen container, as previously described. It is preferable for the coupling projection or coupling protrusion 210 to be formed at an outer circumferential surface of the support unit so as to protrude therefrom or for a lid fastening groove to be formed in the outer circumferential surface of the support unit such that the support unit can be fixed to the cover or lid of the specimen container, in which the specimen collection unit is received, by coupling.

The specimen collection unit 300 is a part that is inserted or introduced into the body of the subject so as to come into contact with a specimen. That is, the specimen collection unit is a part that collects the specimen.

The specimen collection unit 300 is located at a front end of the support unit 200 in order to collect a specimen in the body of the subject that comes into contact with the specimen collection unit. The specimen collection unit 300 includes a screw blade 320 spirally formed along a central axis of the support unit 200 in a longitudinal direction thereof.

That is, the specimen collection unit 300 includes a screw blade 320 configured to allow a specimen brought into contact therewith to be adhered thereto or to be held thereby and to be withdrawn from the body of the subject in that state.

The specimen collection unit may further include a hub 310 coupled to the support unit 200 at the front of the support unit 200, the hub being configured to support the screw blade 320 coupled to or formed on an outer surface thereof. In other words, the hub 310 is coupled to the support unit 200 such that a front portion of the support unit 200 is inserted into the hub, and supports the screw blade 320 disposed on an outer circumferential surface of the hub 310. For reference, the screw blade 320 may be coupled to the front portion of the support unit 200 without the hub 310.

As shown in the figures, at least a part of the screw blade 320 disposed at a central axis of the hub 310 is formed at the hub 310 so as to be coupled thereto. The screw blade 320 may be formed around the hub 310 on an outer circumferential surface of the hub 310. The screw blade 320 thus formed is supported by the hub 310.

The specimen collection unit 300 is connected or coupled to the front of the support unit 200, and is supported by the support unit 200. It is preferable for the specimen collection unit 300 to include at least one screw blade 320 configured to allow a specimen brought into contact therewith to be adhered thereto or to be held thereby and to be withdrawn from the body of the subject in that state and a hub 310 configured to support the screw blade 320.

When the screw blade 320 is included in the specimen collection unit 300, the surface area of the specimen collection unit 300 that contacts the specimen is increased. The hub 310 configured to support the screw blade 320 is coupled to the front portion of the support unit 200. Consequently, the hub 310 supports the screw blade 320 while being supported by the support unit 200.

In the specimen collection unit 300, a gap portion 330 is provided between pitches of the spiral screw blade 320. The gap portion 330 comes into contact with the specimen in the body of the subject.

Each of the screw blades 320 is flexible and elastic. Consequently, each of the plurality of screw blades 320 abuts bodily tissue in the body of the subject. The examiner swings the specimen collection unit 300 in the body of the subject using the handle unit 100 such that the screw blade 320 can scrape or sweep the bodily tissue in which the specimen is present while abutting the bodily tissue. As a result, the specimen is adhered to the surface of the screw blade 320 or is held in a gap between neighboring pitches of the screw blade 320.

That is, the specimen is adhered to the surface of the screw blade 320 or is held in the gap between the neighboring pitches of the screw blade 320 in the state in which the specimen collection unit 300 is inserted in the body of the subject, whereby the specimen remains on the specimen collection unit 300 when the specimen collection unit 300 is withdrawn from the body of the subject. The gap between the neighboring pitches of the screw blade 320 may also be referred to as a collection space or a collection gap.

In the specimen collection unit 300, the specimen brought into contact with the screw blade 320 or the hub 310 may remain held by the screw blade 320 or adhered thereto.

Since the specimen brought into contact with the spiral screw blade 320 disposed around the hub 310 or adhered thereto remains held between the pitches of the screw blade 320 while the specimen is inhibited from being separated from the screw blade 320, as described above, it is possible to further increase the amount of the specimen that is collected, compared to the conventional art.

It is preferable for the screw blade 320 to be manufactured by molding such that the screw blade has a size set depending on the part of the body of the subject into which the collection disks are inserted, such as the nasal cavity, the oral cavity, the anus, or the cervix. The specimen collection unit 300 including the screw blade 320 and the hub 310 may be made of a polymer material that exhibits flexibility and elasticity or an elastomer.

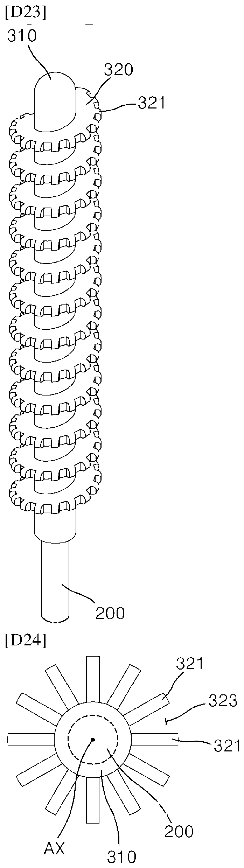

A modified embodiment may also be provided, as shown in FIGs. 22 and 23.

FIG. 22 is a front view schematically showing a modified example of the specimen collection unit of the specimen collection stick according to the third embodiment of the present invention, and FIG. 23 is a perspective view schematically showing the modified example of the specimen collection unit of the specimen collection stick according to the third embodiment of the present invention.

Referring further to FIGs. 22 and 23, it is also preferable for concave and convex parts 321 and 323 to be formed at an outer edge of the screw blade 320, as shown in FIG. 22. In other words, it is also preferable for a plurality of collection recesses 323 to be formed in the outer edge of the screw blade 320. Here, the concave and convex parts 321 and 323 or the collection recesses 323 may have various shapes, and the shape thereof is not limited to a specific shape.

When the plurality of collection recesses 323 or the plurality of concave and convex parts 321 and 323 is formed at the screw blade 320, as described above, the surface area of the outer edge of the screw blade 320 is increased, which assists in increasing the amount of the specimen that is collected.

In the concave and convex parts 321 and 323 of the screw blade 320, the specimen may be held in the collection recesses 323, which are concave portions 323. Here, the specimen may be held due to viscosity of the specimen, elasticity of the flexible screw blade 320, and an increase in surface area of the screw blade 320 that contacts the specimen.

FIGs. 22 and 23 illustratively show an example in which an angularly concave and convex structure is formed at the outer edge of the screw blade; however, the present invention is not limited thereto. For example, curved concave parts and curved convex parts may be alternately formed at the outer edge of the screw blade 320 in the shape of a wave, which is also preferable.

For reference, reference symbol AX indicates the central axis of the support unit 200 or the hub 310.

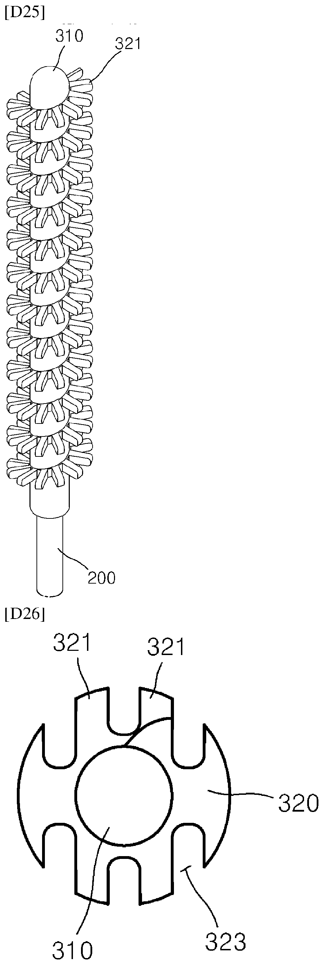

FIG. 24 is a front view schematically showing an applied example of the specimen collection unit of the specimen collection stick according to the third embodiment of the present invention, and FIG. 25 is a perspective view schematically showing the applied example of the specimen collection unit of the specimen collection stick according to the third embodiment of the present invention.

As shown in FIGs. 24 and 25, the screw blade may be partially split (321) at intervals 323. That is, the screw blade may be divided into a plurality of screw blade parts 321 at least partially spaced apart from each other.

Here, the screw blade parts 321, which are divided from each other while being spaced apart from each other, are flexible and elastic, which assists in collecting a specimen with which the screw blade parts come into contact in the body of the subject.

That is, since the divided screw blade parts 321 are flexible and elastic, the specimen may be gathered in a space between the plurality of screw blade parts 321, which is advantageous, and this assists in specimen collection.

When the screw blade 320 is partially split at intervals, as described above, the specimen may be held in the space 323 between the divided parts. Even in this case, the specimen may be held due to viscosity of the specimen, elasticity of the flexible screw blade 320, and an increase in surface area of the screw blade 320 that contacts the specimen.

For reference, reference symbol AX indicates the central axis of the support unit 200 or the hub 310.

Also, it is preferable for a collection recess to be formed in the screw blade as follows.

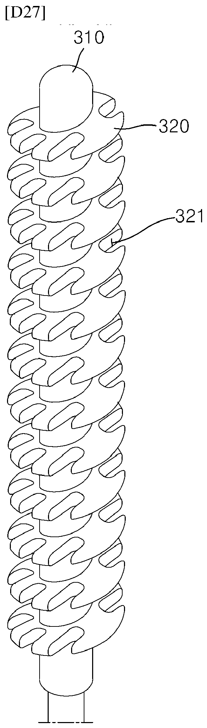

FIG. 26 is a front view schematically showing another modified example of the specimen collection unit of the specimen collection stick according to the third embodiment of the present invention, and FIG. 27 is a perspective view schematically showing the other modified example of the specimen collection unit of the specimen collection stick according to the third embodiment of the present invention.

As shown in FIGs. 26 and 27, a plurality of collection recesses 323 is formed in the outer edge of the screw blade. Also, it is preferable for at least a part 321 of the screw blade 320 to be divided into a plurality of screw blade parts at least partially spaced apart from each other. Alternatively, at least some of the plurality of collection recesses 323 may be formed in a direction perpendicular to the central axis of the hub 310 or the support unit 200 in the longitudinal direction thereof.

Here, at least some of the plurality of collection recesses 323 may be formed in a direction in which neighboring collection recesses 323 are parallel to each other within a predetermined error range. In other words, the longitudinal directions of the formed collection recesses 323 may be parallel to each other.

Even when the plurality of collection recesses 323 is formed in the screw blade 320, as described above, the amount of the specimen that is collected from the body of the subject is increased, in the same manner as the above description.

The structure in which the plurality of collection recesses 323 is formed, as described above, may also be provided, which is also preferable.

In the specimen collection stick according to the third embodiment of the present invention, as described above, the amount of the specimen that is collected by the screw blade 320 included in the specimen collection unit 300 is greater than the amount of the specimen collected by the conventional swab for specimen collection.

Consequently, it is possible to increase the amount of the specimen that is collected from the subject and to increase the amount of the collected specimen that is dissolved or dispersed in a reagent or a solution from the specimen collection stick.

As is apparent from the above description, in the specimen collection stick according to the first embodiment of the present invention, the amount of the specimen collected by the plurality of collection disks included in the specimen collection unit and in the gap between the collection disks is greater than the amount of the specimen collected by the conventional swab for specimen collection.

Also, in the specimen collection stick according to the second embodiment of the present invention, the amount of the specimen that is collected by the plurality of collection blades included in the specimen collection unit and in the gap between neighboring ones of the collection blades is greater than the amount of the specimen collected by the conventional swab for specimen collection.

Furthermore, in the specimen collection stick according to the third embodiment of the present invention, the amount of the specimen that is collected by the screw blade included in the specimen collection unit is greater than the amount of the specimen collected by the conventional swab for specimen collection.

As can be seen from the above description, the specimen collection stick according to the present invention has advantages in that a flocking process, which is required to manufacture the conventional swab for specimen collection, is not necessary, whereby it is possible to reduce the manufacturing cost of the specimen collection stick, and in that there is no fiber layer in the specimen collection stick, whereby it is possible to prevent a problem in which the fiber layer is separated from the specimen collection stick and remains in the body of the subject, unlike the conventional swab for specimen collection.

In addition, the specimen collection stick according to the present invention has advantages in that it is possible to increase the amount of the specimen that is collected from the subject, compared to the conventional swab, to increase the amount of the collected specimen that is dissolved or dispersed in a reagent or a solution from the specimen collection stick, and to improve examination accuracy with an increase in amount of the specimen that is collected.

Furthermore, the specimen collection stick according to the present invention has advantages in that it is possible to shorten time necessary for specimen collection and to reduce inconvenience of the subject during specimen collection. In addition, the specimen collection unit, which is inserted into the oral cavity or the nasal cavity of the subject, is made of a polymer material that exhibits flexibility and elasticity or an elastomer material, whereby it is possible to reduce inconvenience of the user.

Although the present invention has been described in detail based on the embodiment with reference to the accompanying drawings, as described above, the embodiments are merely provided to describe the present invention, and therefore it should be understood that the present invention is not limited to the embodiments, and the scope of rights of the present invention should be interpreted by the following claims and equivalent thereto.
100: Handle unit
200: Support unit
300: Specimen collection unit
310: Hub
320: Collection blade, screw blade

## Claims

1. A specimen collection stick comprising:
a rod-shaped or bar-shaped support unit having a predetermined length; and
a specimen collection unit located at a front end of the support unit, the specimen collection unit being configured to collect a specimen in a body of a subject that comes into contact with the specimen collection unit, the specimen collection unit comprising a plurality of collection disks arranged in a longitudinal direction of the support unit.

2. The specimen collection stick according to claim 1, wherein the specimen collection unit further comprises a hub coupled to the support unit such that a front portion of the support unit is inserted into the hub, the hub being configured to support the plurality of collection disks.

3. The specimen collection stick according to claim 2, wherein
each of the collection disks is formed in a plate shape having at least one curved or flat side surface, and
an edge of each of the collection disks is circular or oval.

4. The specimen collection stick according to claim 3, wherein
the plurality of collection disks is coupled to or formed at the hub such that the hub extends through a central part of each of the plurality of collection disks, and
a predetermined gap is provided between neighboring ones of the collection disks such that the specimen can remain in the gap.

5. The specimen collection stick according to claim 4, wherein a coupling recess or coupling hole is formed in the hub such that the front portion of the support unit is inserted into the coupling recess or coupling hole, whereby the front portion of the support unit is coupled to the hub.

6. The specimen collection stick according to claim 5, wherein
the hub is provided in an outer surface thereof with a support unit fastening recess or support unit fastening hole configured to communicate with the coupling recess or coupling hole of the hub, and
a hub fastening projection or hub fastening protrusion coupled to the support unit fastening recess or support unit fastening hole by fitting as the front portion of the support unit is inserted into the coupling recess or coupling hole is formed on an outer surface of the front portion of the support unit.

7. The specimen collection stick according to claim 4, wherein, in at least some of the plurality of collection disks, neighboring ones of the collection disks have different diameters or different long radii.

8. The specimen collection stick according to claim 1, further comprising a handle unit connected to a rear end of the support unit, the handle unit having a predetermined length such that the handle unit can be gripped by a hand or fingers of an examiner.

9. The specimen collection stick according to claim 8, wherein a segment groove is provided between the handle unit and the support unit such that the handle unit and the support unit can be separated from each other.

10. The specimen collection stick according to claim 1, wherein a coupling projection or coupling protrusion is formed at an outer circumferential surface of the support unit so as to protrude therefrom, or a lid fastening groove is formed in the outer circumferential surface of the support unit, such that the support unit can be fixed to a lid of a specimen container configured to receive the specimen collection unit therein by coupling.

11. A specimen collection stick comprising:
a rod-shaped or bar-shaped support unit having a predetermined length; and
a specimen collection unit located at a front end of the support unit, the specimen collection unit being configured to collect a specimen in a body of a subject that comes into contact with the specimen collection unit, the specimen collection unit comprising a plurality of collection blades disposed in rotational symmetry or axial symmetry with respect to a central axis of the specimen collection unit in a longitudinal direction thereof.

12. The specimen collection stick according to claim 11, wherein the specimen collection unit further comprises a hub coupled to the support unit such that a front portion of the support unit is inserted into the hub, the hub being configured to support the plurality of collection blades.

13. The specimen collection stick according to claim 12, wherein each of the collection blades is formed in a plate shape having at least one curved or flat side surface.

14. The specimen collection stick according to claim 13, wherein a length of an inner side of each of the collection blades abutting the hub by coupling is equal to or less than a length of an outer side thereof.

15. The specimen collection stick according to claim 12, wherein
the plurality of collection blades arranged at an outer circumferential surface of the hub on the same circumference constitute a blade group, and
a plurality of neighboring blade groups is disposed on the outer circumferential surface of the hub such that each of the blade groups is spaced apart from a corresponding one of the blade groups neighboring thereto by a predetermined distance.

16. The specimen collection stick according to claim 11, further comprising a handle unit connected to a rear end of the support unit, the handle unit having a predetermined length such that the handle unit can be gripped by a hand or fingers of an examiner.

17. The specimen collection stick according to claim 16, wherein a segment groove is provided between the handle unit and the support unit such that the handle unit and the support unit can be separated from each other.

18. The specimen collection stick according to claim 11, wherein a coupling projection or coupling protrusion is formed at an outer circumferential surface of the support unit so as to protrude therefrom, or a lid fastening groove is formed in the outer circumferential surface of the support unit, such that the support unit can be fixed to a lid of a specimen container configured to receive the specimen collection unit therein by coupling.

19. The specimen collection stick according to claim 12, wherein each of the collection blades or the hub is made of a flexible or elastic material.

20. A specimen collection stick comprising:
a rod-shaped or bar-shaped support unit having a predetermined length; and
a specimen collection unit located at a front end of the support unit, the specimen collection unit being configured to collect a specimen in a body of a subject that comes into contact with the specimen collection unit, wherein
the specimen collection unit comprises:
a collection blade having a plate shape, the collection blade being configured to inhibit separation of the specimen that comes into contact with the specimen collection unit; and
a hub coupled to the support unit such that a front portion of the support unit is inserted into the hub, the hub being configured to support a plurality of collection blades disposed on an outer circumferential surface thereof, and
the plurality of collection blades disposed on the outer circumferential surface of the hub is disposed asymmetrically with respect to a central axis of the hub or the support unit in a longitudinal direction thereof.

21. A specimen collection stick comprising:
a rod-shaped or bar-shaped support unit having a predetermined length; and
a specimen collection unit located at a front end of the support unit, the specimen collection unit being configured to collect a specimen in a body of a subject that comes into contact with the specimen collection unit, the specimen collection unit comprising a screw blade spirally formed along a central axis of the support unit in a longitudinal direction thereof.

22. The specimen collection stick according to claim 21, wherein the specimen collection unit further comprises a hub coupled to the support unit such that a front portion of the support unit is inserted into the hub, the hub being configured to support the screw blade disposed on an outer circumferential surface thereof.

23. The specimen collection stick according to claim 22, wherein a plurality of collection recesses is formed in an outer edge of the screw blade.

24. The specimen collection stick according to claim 23, wherein at least some of the plurality of collection recesses are formed in a direction perpendicular to a central axis of the hub or the support unit in a longitudinal direction thereof.

25. The specimen collection stick according to claim 23, wherein at least some of the plurality of collection recesses are formed in a direction in which neighboring collection recesses are parallel to each other within a predetermined error range.

26. The specimen collection stick according to claim 22, wherein at least a part of the screw blade is divided into a plurality of screw blade parts at least partially spaced apart from each other.

27. The specimen collection stick according to claim 22, wherein the screw blade or the hub is made of a flexible or elastic material.

28. The specimen collection stick according to claim 21, further comprising a handle unit connected to a rear end of the support unit, the handle unit having a predetermined length such that the handle unit can be gripped by a hand or fingers of an examiner.

29. The specimen collection stick according to claim 28, wherein a segment groove is provided between the handle unit and the support unit such that the handle unit and the support unit can be separated from each other.

30. The specimen collection stick according to claim 21, wherein a coupling projection or coupling protrusion is formed at an outer circumferential surface of the support unit so as to protrude therefrom, or a lid fastening groove is formed in the outer circumferential surface of the support unit, such that the support unit can be fixed to a lid of a specimen container configured to receive the specimen collection unit therein by coupling.
